(19) Europäisches Patentamt
European Patent Office
Office européen des brevets

(11) Publication number: 0 403 543 B1

# (12) EUROPEAN PATENT SPECIFICATION

(45) Date of publication of patent specification: 04.08.93

(51) Int. Cl.[5]: C07C 69/76, C08G 63/52, C08G 63/66

(21) Application number: 89903653.7

(22) Date of filing: 28.02.89

(86) International application number: PCT/US89/00797

(87) International publication number: WO 89/08097 (08.09.89 89/21)

## (54) POLYFUNCTIONAL VINYL ETHER TERMINATED ESTER OLIGOMERS.

(30) Priority: 29.02.88 US 161823

(43) Date of publication of application: 27.12.90 Bulletin 90/52

(45) Publication of the grant of the patent: 04.08.93 Bulletin 93/31

(84) Designated Contracting States: BE CH DE FR GB LI LU NL SE

(56) References cited:
GB-A- 1 225 059
US-A- 4 749 807

(73) Proprietor: ALLIED-SIGNAL INC.
Columbia Road and Park Avenue P.O. Box 2245R
Morristown New Jersey 07962-2245(US)

(72) Inventor: LAPIN, Stephen, C.
26478 Northeast Lakeshore Drive
Wauconda, IL 60084(US)
Inventor: OLIVARES, Jorge, M.
2501 Soldiers Home Rd 58C
West Lafayette, IN 47906-1758(US)

(74) Representative: Brock, Peter William et al
UROUHART-DYKES & LORD 91 Wimpole Street
London W1M 8AH (GB)

EP 0 403 543 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Vinyl ethers are extremely reactive monomers which are known to undergo polymerization by a cationic mechanism and are useful in applications which require a high speed curing of a resin formulation. The vinyl ethers react much faster than the epoxy resins and therefore may be used for printing inks, coatings, elastomers, foams, and other types of products dependent upon the ability of the resin to cure at a rate which is consistent with other processing steps. A disadvantage attendant to the use of vinyl ethers is that their commercial availability is relatively limited. In general, the available vinyl ethers are low molecular weight monofunctional or difunctional monomers, whereas in most commercial applications higher molecular weight oligomeric materials are preferred.

The present invention discloses vinyl ether terminated esters. As will be seen, the structure of such esters is susceptible to wide variations with a minimum change in the reactants. This flexibility permits facile variation in the properties and characteristics of the vinyl ether terminated ester oligomers as well as comparable variations in the resulting cured resins. Where the oligomer contains more than one vinyl ether group the cured resins are extensively cross-linked, very high molecular weight polymers. The polymers are thermosetting materials with a wide range of properties depending upon the structure of the oligomeric precursor. Although the vinyl ether terminated esters of this invention have been designed to fill the need for radiation curable coatings, they may have a much broader use. In particular, the esters of our invention are readily polymerized by means other than radiation curing, and the resulting polymers are meant to be subsumed in our invention.

## SUMMARY OF THE INVENTION

The purpose of our invention is to provide vinyl ethers which are readily and economically synthesized and with structures whose permutations are large in number but each of which are easily made, with at least most of the resulting materials able to be radiation cured to afford polymer coatings. An embodiment is the class of polyfunctional vinyl ether terminated oligomeric esters where the acid portion of the ester is a polycarboxylic acid which is at least a tricarboxylic acid. In a more specific embodiment the alcohol portion of the ester is a vinyl ether which can be viewed as the adduct of an acetylenic compound and an ethylene or propylene glycol or a bis(hydroxy)cycloalkane. In another embodiment the alcohol portion of the ester is a vinyl ether which may be viewed as the adduct of an acetylenic compound and a poly(ethylene) or poly-(propylene) glycol. In a further embodiment the acetylenic compound is a terminal acetylene. In a still further embodiment the acid portion of the ester is a tricarboxylic acid. In yet another specific embodiment a glycol is used as a chain extender for the oligomeric ester. Other embodiments will become apparent from the detailed discussion within.

The present invention provides a vinyl ether terminated oligomeric ester containing fewer than 5% unreacted alcoholic hydroxyl groups, which is the esterification reaction product of a vinyl ether terminated alcohol of the formula $R_1CH=CR_2OXOH$, or of a mixture of alcohols containing 1 molar proportion of the vinyl ether terminated alcohol of the aforesaid formula and up to 100 molar proportions of at least one diol of formula $Z(OH)_2$, with a sufficient amount of a polycarboxylic acid having at least three carboxyl moieties, such that there are essentially no free alcoholic hydroxyl groups in the resulting ester, wherein

$R_1$ and $R_2$ are independently selected from hydrogen and alkyl having up to 10 carbon atoms; and X and Z are independently selected from alkylene of the formula $C_nH_{2n}$, where n is an integer from 2 to 10;

poly(ethyleneoxy) or poly(propyleneoxy) having the formula

$[-CH_2CH_2O]_m - CH_2CH_2 -$ or 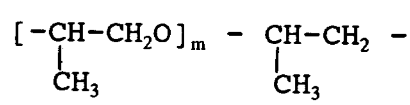

respectively, where m is a number from 0 to 49, and a cycloaliphatic group of the formula $[-(CH_2)_n]_2R_3$ where n is an integer from 1 to 6, and $R_3$ is a cycloalkylene group whose ring is a saturated ring having 5 to 8 carbons.

The invention to be described in great detail within provides a class of compounds with a broad spectrum of molecular weight but which is characterized by the presence of one or more terminal vinyl ether moieties and which are esters of polycarboxylic acids. One of the reactants used in making the products of our invention is, or may be viewed as, an adduct of an acetylenic compound (an alkyne) with a

diol, the resulting material being a vinyl ether terminated alcohol. For simplicity, a generic diol, HOXOH, will be used in this section as representative of diols generally, in order to represent one means of preparing the vinyl ether terminated alcohol under discussion, namely,

$R_1C=CR_2$ + HOXOH -> $R_1CH\equiv CR_2OXOH$.

If the vinyl ether terminated alcohol is prepared as an adduct, reaction conditions usually are so chosen as to form the monoadduct either to the virtual exclusion of the diadduct or, more likely, in large preponderance relative to the diadduct. The monoadduct can be isolated and used in a purified form, but more often the entire reaction mixture is used as the alcoholic reactant in ester formation with carboxylic acids, where the unreacted glycol (or diol) has the important function of a chain extender.
The vinyl ether terminated alcohol is then reacted with a polycarboxylic acid. In reality, reaction of the alcohol and carboxylic acid is too slow or too incomplete for commercial preparation, and the alcohol is reacted with some activated derivative of a carboxylic acid, such as an acid chloride or anhydride. But for simplicity and clarity of exposition we shall continue to refer to reaction with a carboxylic acid. In the case where the acid is a tricarboxylic acid the reaction may be represented as,

$3R_1CH=CR_2OXOH + [HO(O)C]_3Y \rightarrow [R_1CH=CR_2OXO(O)C]_3Y$

The above reaction is most accurate where the alcoholic reactant is solely

$R_1CH=CR_2OXOH$,

and in the more usual cases where the alcoholic reactant is a mixture containing unreacted glycol, or where a second glycol is added to the vinyl ether terminated alcohol, the product is a mixture of oligomeric esters.

Such oligomeric esters, which arise in a more-or-less typical condensation reaction of a polyfunctional carboxylic acid acid and glycol, can be envisioned as arising from multiple consecutive esterification reactions where the polycarboxylic acids first react with the glycol to form oligomeric polycarboxylic acids. The latter then can be end-capped by esterification with a vinyl ether terminated alcohol to afford the oligomeric product. The oligomeric esters need not arise from the precise reaction sequence described above, for other sequences can lead to the same oligomeric esters. It will be readily appreciated that because of the polyfunctionality of the carboxylic acids used, the products will be a complex mixture differing in molecular weight, in relative number of vinyl ether groups, and so forth.

The vinyl ether terminated alcohols which are used in preparing the oligomeric esters of this invention have a structure corresponding to the adduct of an alkyne and a diol. In this application the terms "diol" and "glycol" are used interchangeably and meant to be equivalent. It must be emphasized that although some of the vinyl ether terminated alcohols of this invention may in fact be made by the addition of diols to alkynes, the vinyl ether terminated alcohols herein also can be made in other ways, and the alternative routes to such alcohols are often preferred. The alkyne has the generic formula $R_1C\equiv CR_2$, and the diol has the generic formula $X(OH)_2$. The generic formula of the vinyl ether terminated alcohols of our invention is then $R_1CH=CR_2OXOH$.

The groupings $R_1$ and $R_2$ are independently selected from the group consisting of hydrogen and lower alkyl moieties containing from 1 to 10 carbon atoms, although those with from 1 to about 4 carbon atoms are favored. It is preferable that both $R_1$ and $R_2$ are not alkyl moieties, for in the case where both are lower alkyl groups this causes a reduction in polymerization rate of the oligomers of our invention to a point where the polymerization rate is undesirable. Where $R_1$ is an alkyl moiety it is preferred that $R_2$ be hydrogen, and conversely; those cases where $R_2$ is hydrogen and $R_1$ an alkyl of 1 to 4 carbons are quite desirable. In a preferred embodiment $R_1(R_2)$ is a methyl group and $R_2(R_1)$ is hydrogen. In a still more preferred embodiment both $R_1$ and $R_2$ are hydrogen.

In the vinyl ether alcohol fragment, the grouping -OXOH arises from, or can be thought of as arising from, a diol of structure $X(OH)_2$. Among the diols HOXOH supplying the necessary grouping in the vinyl ether alcohol fragment one important class consists of alkylene glycols, $HO(C_nH_{2n})OH$, where n is an integer from 2 to 10. The linear alkylene glycols, $HO(CH_2)_nOH$, (polymethylenediols), where n is an integer from 2 to 10, are particularly useful, especially where n is from 2 to 6. Illustrative of the members of this group are such diols as ethylene glycol, 1,3-propylene glycol, 1,4-butanediol, 1,5-pentanediol, 1,6-hexanediol, 1,7-heptanediol, 1,8-octanediol, 1,9-nonanediol, and 1,10-decanediol (decamethylene glycol).

The nonlinear or branched alkylene diols also may be used to supply the fragment -OXOH, where such glycols contain from 3 up to 10 carbon atoms. Examples include 1,2-propylene glycol, 2,3-butanediol, 2,3-dimethyl-2,3-butanediol, 2,3-dimethyl-1,4-butahediol, etc.

Another class of diols useful as a source of the grouping -OXOH are the polyalkyleneoxy glycols, especially poly(ethyleneoxy) glycols, $[-CH_2CH_2O-]_m$, and poly(propyleneoxy) glycols, $[-CH(CH_3)CH_2O-]_m$, where m is from 1 to 50, although more usually m is 1 to 10, and most preferably from 1 to 5. Examples of the glycols in this branch of the invention include diethylene glycol, triethylene glycol, tetraethylene glycol, pentaethylene glycol, hexaethylene glycol, etc., along with the analogs of the propyleneoxy glycols.

Yet another class of diols used in the practice of this invention are bis(hydroxyalkyl)cycloalkanes whose formula is $(HO(CH_2)_n)_2R$. It is to be noted that in all cases the hydroxyl moiety is a primary hydroxyl, i.e., it is located at the end of the alkylene chain. In the diols of this branch of our invention n is an integer from 1 to 6, preferably from 1 to 3, and the member where n = 1 is highly preferred. R is a divalent radical whose parent is a cyclopentane, cyclohexane, cycloheptane, or cyclooatane, with the cyclohexanes being favored because of their relative availability.

Examples of the aforementioned diols include bis(hydroxymethyl)cyclopentane, bis(2-hydroxyethyl)-cyclopentane, bis(3-hydroxypropyl)cyclopentane, bis(4-hydroxybutyl)cyclopentane, bis(5-hydroxypentyl)-cyclopentane, bis(6-hydroxyhexyl)cyclopentane, bis(hydroxymethyl)cyclohexane, bis(2-hydroxyethyl)-cyclohexane, bis(3-hydroxypropyl)cyclohexane, bis(4-hydroxybutyl)cyclohexane, bis(5-hydroxypentyl)-cyclohexane, bis(6-hydroxyhexyl)cyclohexane, and the cycloheptane and cyclooctane analogs of the foregoing diols.

As regards the orientation of the hydroxy alkyl groups, the preferred members are the 1,3-bis-(hydroxyalkyl)cyclopentanes and the 1,4-bis(hydroxyalkyl)cyclohexanes, -cycloheptanes, and -cyclooctanes. Diols substituted at positions different from those specified above may be used in the practice of this invention, but not necessarily with equivalent results. The bis(hydroxymethyl)cyclohexanes are a highly preferred diol used in the practice of this invention as they are readily available from the reduction of the corresponding phthalic acids, and among these 1,4-bis(hydroxymethyl)cyclohexane is greatly favored.

The vinyl ether terminated alcohol is then reacted with a polycarboxylic acid which is at least a tricarboxylic acid to afford a vinyl ether terminated ester. There are four quite distinct variants here, three of which utilize diol $Z(OH)_2$ as a reactant. In one variant the purified vinyl ether terminated alcohol alone is reacted with the acid. In a second variant a mixture of the vinyl ether terminated alcohol and the unreacted diol from which it was made, or could be thought of as being made, is reacted with the acid. In this variant the diol, $Z(OH)_2$, acts as a chain extender by esterifying the polycarboxylic acid to give an oligomeric ester where $X=Z$. In another variant a mixture of the vinyl ether terminated alcohol and a second diol, $Z(OH)_2$, is reacted with the acid. Again the diol acts as a chain extender via ester formation with the carboxylic acid, but in this case X and Z are different. Finally, in a fourth variant a mixture of vinyl ether terminated alcohol, unreacted diol from which it was made or could be thought to be made, and a second, unrelated diol is reacted with the polycarboxylic acid. As in the cases above, the unreacted diols react with the carboxylic acid to afford oligomeric esters, and in this variant some of Z are different from X and some are the same.

The components of the alcohol mixture reacting with the carboxylic acid are $R_1CH=CR_2OX(OH)$ (component A), $X(OH)_2$ (component B), and $Z(OH)_2$ (component C), where $Z(OH)_2$ is selected from the same group as $X(OH)_2$, but merely denotes a different member of that group. In the reactant alcohol mixture the molar proportions of $(B+C)/A$ may be between 0 and 100. Where the ratio is 0 there is no free diol which may be a common and desirable case, but generally the alcohol mixture will contain not only a vinyl ether terminated alcohol but also some diol. In the preferred case the ratio above is between 0 and 5.

As alluded to above, the reaction between alcohols and carboxylic acids to give the esters of this invention is too slow and occasionally too incomplete to be a practical method of preparation, and activated derivatives of carboxylic acids are in fact used as reactants. Among such derivatives the acid chlorides and anhydrides are most frequently employed, and the following description refers to the carboxylic acids which are parents of the activated acid derivatives used to prepare our oligameric esters.

Turning to the polycarboxylic acids which may be used, or which are parents of activated derivatives which may be used in the practice of this invention, it has previously been mentioned that at least tricarboxylic acids are necessary. Among the tricarboxylic acids which represent the acid portion of the vinyl ether terminated esters of this invention may be mentioned the benzenetricarboxylic acids, especially 1,3,5-benzenetricarboxylic acid (trimesic acid), 1,2,4-benzenetricarboxylic acid (trimellitic acid), their reduced hexahydro counterparts, and citric acid. Among the tetracarboxylic acids may be mentioned the benzenetetracarboxylic acids, especially 1,2,4,5-benzenetetracarboxylic acid (pyromellitic acid), 3,3',4,4'-benzophenonetetracarboxylic acid, 1,4,5,8-naphthalenetetracarboxylic acid, their reduced hexahydro counterparts, and bicyclo [2.2.1]heptane-2,3,5,6-tetracarboxylic acid.

Where the alcohol mixture contains 1 or more diols there are 3 subvariants possible. In all cases the alcohol mixture contains at least one vinyl ether terminated alcohol, and the subvariants are the cases

where the mixture also contains the diol containing the structural grouping found in the ether, an unrelated diol, or a mixture of the above. In either of these cases, the ratio of molar proportions of diol to vinyl ether terminated alcohol is up to 100, and preferably is from 0 to 5.

As previously stated, the structure of the products of this invention is capable of enormous permutation. For example, each of the hydroxyls of the diol can react with a different oligomeric ester subunit arising from the reaction of a polycarboxylic acid and a diol. Also, in any subunit of the oligomeric ester a diol may react intermolecularly with various carboxyl groups to afford extensively cross-linked subunits. An important characteristic of our invention is that in all cases there are essentially no free hydroxyl groups arising from the diol in the final product. That is, less than about 5% of the initial hydroxyl groups of the diols remain unreacted. This is desirable to afford a polymer with good characteristics.

The vinyl ether terminated oligomeric esters of this invention may be cured or polymerized by any method known in the art. For example, the resins may be radiation cured, as for example by being subjected to an electron beam of an energy in the range from 50 up to perhaps 500 KeV with a dosage from 0.1 to 10.0 Mrads. Electron beam curing may be performed advantageously in the presence of an iodonium or a sulfonium salt to afford high speed cationic polymerization. Ultraviolet curing in the presence of an onium salt also may be executed to afford cationic polymerization. Other means include thermal curing in the presence of a Lewis acid, such as boron trifluoride, or in the presence of a strong acid such as p-toluenesulfonic acid and trifluoromethylsulfonic acid. All these methods of polymerization are well known to those versed in the art and need not be elaborated upon further.

The high crosslinking density of the products of our invention affords polymeric coatings with excellent solvent resistance. Increasing crosslinking density often is accompanied by harder, less flexible coatings with lower elongation and impact resistance, and where these properties are not desired crosslink density may be reduced by diluting the products of our invention with, for example, vinyl ethers of lower crosslinking density, such as vinyl ethers of various monofunctional and difunctional alcohols. Monofunctional vinyl ethers are best for reducing crosslink density, and illustrative of the readily available vinyl ethers are isooctyl vinyl ether, decyl vinyl ether, hexadecyl vinyl ether, octadecyl vinyl ether, n-butyl vinyl ether, and isobutyl vinyl ether. In general, the vinyl ether of any alkanol or cycloalkanol may be used in the practice of this aspect of my invention. Among the vinyl ethers of difunctional alcohols may be mentioned triethylene glycol divinyl ether, diethylene glycol divinyl ether, 1,4-cyclohexane dimethanol divinyl ether, and 1,6-hexanediol divinyl ether as exemplary of this class.

The following examples are only illustrative of our invention which is not to be limited thereto or circumscribed thereby in any way.

EXAMPLE 1

**Synthesis of Oligomeric Ester Based on 1,3,5-Benzenetricarboxylic (Trimesic) Acid.** Triethyleneglycol monovinyl ether, (TEGMVE, 20g, 0.114 moles), triethylamine (14g) and dimethylaminopyridine (0.2g) were combined with 150 mL of anhydrous ether in 250 mL round bottom flask fit with an addition funnel, condenser and stirrer. The addition funnel was charged with trimesoyl chloride (10.0g, .038 mol) in 70 mL of ether. The acid chloride solution was added dropwise over a 40 min. period. Solid triethylamine hydrochloride precipitated from the reaction mixture. The mixture was filtered and then washed two times with 300 mL of 2% $H_3PO_4$ followed by 5% $NaHCO_3$ and then water. The ether layer was dried over $Na_2SO_4$ and was then concentrated in a vacuum to afford 16.22 g (60% yield) of a clear, light yellow liquid, A. The HNMR was consistent with the expected product.

The procedure was repeated using 1,4-butanediol monovinyl ether, BDMVE, in place of TEGMVE to produce an analogous ester B.

EXAMPLE 2

**Radiation Curing.** Samples to be irradiated were combined with a triarylsulfonium salt (General Electric UVE-1016, 2 weight percent) and were coated onto either Bonderite-40 treated steel test panels (Parker Chemical) or polyethylene coated paper board. An excess of the sample was placed at one end of the substrate and a #6 wire wound rod was drawn across the substrate with even pressure pushing excess material off the edge. This method produced coatings with a thickness of 6 to 12$\mu$m.

An RPC model QC-1202 processor was used for UV curing. The unit was equipped with two 25.4cm (12 inch) medium pressure mercury arc lamps and a variable speed conveyor at 0.25 to 2.54 m/s (50 to 500 ft/min). Only one lamp was used at a time in the testing operated at 78.75 w/cm (200 watts/in).

An Energy Sciences Electrocurtain model CB-150 equipped with a 15 cm linear cathode was used for EB curing. Electron energies of 160 KeV were employed. Samples were placed in an aluminum tray on a variable speed conveyor at 0.1 to 1.2 m/s (20-235 ft/min) within the CB-150 unit. Irradiation occurred in a nitrogen atmosphere.

The coatings were evaluated within one hour after irradiation. The coatings were examined for solvent resistance using methyl ethyl ketone. The number of double rubs necessary to break through the coating was recorded. Reverse impact was measured on the steel panels using a Gardner impact tester according to ASTM Method D2794. The coating elongation was measured by bending the coated steel panel over a conical mandrel according to ASTM Method D522. Adhesion was measured according to ASTM D3359 using Scotch 610 adhesive tape. Pencil hardness was measured according to ASTM D3363.

**Table 1. Properties of UV Cured[a] Coatings**

| VEE | Max. Conveyor Speed for Tack-free Cure m/s (ft/min) | MEK Double Runs[c] | Adhesion PE/Treated Steel (%) | Pencil Hardness[c] | Elongation[c] (%) | Reverse Impact[c] J (in-lbs) |
|---|---|---|---|---|---|---|
| A | >2.54 (>500[b]) | >200 | 100/0 | 3H | 18 | 5.65 (50) |
| B | >2.54 (>500[b]) | >200 | 100/0 | 2H | 9 | 0.45 (4) |

a. One 78.75 W/cm (200 watt/in.) medium pressure mercury arc Lamp.

b. **Maximum speed of curing unit.**

c. After curing at 1.27 m/s (250 ft/min.)

Table 2. Properties of EB Cured Coatings

| VEE | Min. EB Dose for Tack-free Cure (Mrads) | MEK* Double Rubs | Adhesion* PE/Treated Steel (%) | Pencil* Hardness | Elongation* (%) | Reverse Impact J (in-lbs) |
|---|---|---|---|---|---|---|
| A | 0.50 | 70 | 40/0 | 2H | 20 | 3.39 (30) |
| B | 0.75 | > 200 | 100/4 | 3H | 8 | 0.45 (4) |

* After EB curing at 4.0 Mrads.

EXAMPLE 3

**Preparation of an Oligomeric Multifunctional Vinyl Ether Ester.**

A higher molecular weight material may be prepared by including a difunctional alcohol in the reaction mixture. Thus, diethylene glycol (10.5 g, 0.1 mol) and diethylene glycol monovinyl ether (DEGMVE, 52.4 g, 0.4 mol) may be combined with triethylamine (60 g) and dimethylaminopyridine (0.8 g) along with 250 mL of anhydrous ether in a one liter round bottom flask fit with an addition funnel, condenser and stirrer. The addition funnel may be charged with trimesoyl chloride (53.1 g, 0.2 mol) in 200 mL of ether. The acid chloride solution may be added dropwise over a 1.0 hour period. When reaction is complete, the mixture is filtered and then washed two times with 1.0 L of 2% $H_3PO_4$ followed by 5% $NaHCO_3$ and then water. The products may be obtained after evaporating the ether in a vacuum. The product is a mixture of various molecular weight oligomers, and may be combined with an appropriate onium salt catalyst and cured by UV or EB irradiation.

## Claims

1. A vinyl ether terminated oligomeric ester containing fewer than 5% unreacted alcoholic hydroxyl groups, which is the esterification reaction product of a vinyl ether terminated alcohol of the formula $R_1CH=CR_2OXOH$, or of a mixture of alcohols containing 1 molar proportion of the vinyl ether terminated alcohol of the aforesaid formula and up to 100 molar proportions of at least one diol of formula $Z(OH)_2$, with a sufficient amount of a polycarboxylic acid having at least three carboxyl moieties, such that there are essentially no free alcoholic hydroxyl groups in the resulting ester, wherein

   $R_1$ and $R_2$ are independently selected from hydrogen and alkyl having up to 10 carbon atoms; and X and Z are independently selected from alkylene of the formula $C_nH_{2n,}$ where n is an integer from 2 to 10;

   poly(ethyleneoxy) or poly(propyleneoxy) having the formula

$$[-CH_2CH_2O]_m - CH_2CH_2 - \text{ or } [-\underset{CH_3}{\underset{|}{C}}H-CH_2O]_m - \underset{CH_3}{\underset{|}{C}}H-CH_2 -$$

   respectively, where m is a number from 0 to 49, and a cycloaliphatic group of the formula $[-(CH_2)_n]_2R_3$ where n is an integer from 1 to 6, and $R_3$ is a cycloalkylene group whose ring is a saturated ring having 5 to 8 carbons;

2. An oligomer according to Claim 1 characterised in that X is alkylene of the formula $-(CH_2)_n-$, where n is an integer from 1 to 4.

3. An oligomer according to Claim 1 characterised in that X is poly(ethyleneoxy) or poly(propyleneoxy) of the formula set out in Claim 1 where m is from 1 to 5.

4. An oligomer according to Claim 1 characterised in that X is a group of the formula $-CH_2-R_3-CH_2-$ wherein $R_3$ is cyclopentylene, cyclohexylene, cycloheptylene or cyclooctylene.

5. The vinyl ether terminated oligomer of Claim 1 where the polycarboxylic acid is a tricarboxylic acid.

6. The vinyl ether terminated oligomer of Claim 1 where the polycarboxylic acid is a tetracarboxylic acid.

7. A polymeric product obtainable by polymerizing the vinyl ether terminated oligomer according to any one of Claims 1 to 6.

8. A polymeric product according to Claim 7 characterised in that the vinyl ether terminated oligomer contains a divinyl ether.

## Patentansprüche

1. Esteroligomer mit endständigem Vinyläther und weniger als 5% unumgesetzten alkoholischen Hydroxylgruppen, welches das Veresterungs-Reaktionsprodukt eines Alkohols mit endständigem Vinyläther von der Formel $R_1CH=CR_2OXOH$ oder einer Mischung von Alkoholen, die 1 Molanteil des Alkohols mit endständigem Vinyläther der zuvor erwähnten Formel und bis zu 100 Molanteile wenigstens eines Diols der Formel $Z(OH)_2$ enthalten, mit einer ausreichenden Menge einer Polycarbonsäure mit zumindest drei Carboxylhälften ist, so daß in dem sich ergebenden Ester im wesentlichen keine freien alkoholischen Hydroxylgruppen vorhanden sind, in welchen Formeln

   $R_1$ und $R_2$ unabhängig voneinander aus Wasserstoff und Alkylgruppen mit bis zu 10 Kohlenstoffatomen gewählt sind; und X und Z unabhängig voneinander aus Alkylen der Formel $C_nH_{2n}$, worin n eine ganze Zahl von 2 bis 10 ist;

   jeweils aus Poly(Äthylenoxy) oder Poly (Propylenoxy)mit der Formel

$$[-CH_2CH_2O]_m - CH_2CH_2 -$$ oder 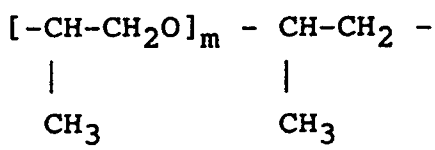

   worin m eine Zahl von 0 bis 49 ist, und einer cycloaliphatischen Gruppe der Formel $[-CH_2)_n]_2R_3$ gewählt ist, worin n eine ganze Zahl von 1 bis 6 und $R_3$eine Cycloalkylengruppe ist, deren Ring ein gesättigter Ring mit 5 bis 8 Kohlenstoffen ist;

2. Oligomer nach Anspruch 1, dadurch gekennzeichnet, daß X ein Alkylen der Formel $-(CH_2)_n-$ ist, worin n eine ganze Zahl von 1 bis 4 ist.

3. Oligomer nach Anspruch 1, dadurch gekennzeichnet, daß X ein Poly(Äthylenoxy) oder ein Poly-(Propylenoxy) der in Anspruch 1 angegebenen Formel ist, worin m 1 bis 5 ist.

4. Oligomer nach Anspruch 1, dadurch gekennzeichnet, daß X eine Gruppe der Formel $-CH_2-R_3-CH_2-$ ist, worin $R_3$ Cyclopentylen, Cyclohexylen, Cycloheptylen oder Cyclooctylen ist.

5. Esteroligomer mit endständigem Vinyläther nach Anspruch 1, worin die Polycarbonsäure eine Tricarbonsäure ist.

6. Esteroligomer mit endständigem Vinyläther nach Anspruch 1, worin die Polycarbonsäure eine Tetracarbonsäure ist.

7. Ein durch Polymerisieren des Esteroligomers mit endständigem Vinyläther nach einem der Ansprüche 1 bis 6 erhältliches Polymerprodukt.

8. Polymerprodukt nach Anspruch 7, dadurch gekennzeichnet, daß das Oligomer mit endständigem Vinyläther einen Divinyläther enthält.

## Revendications

1. Ester oligomérique à vinyle éther terminal contenant moins de 5% de groupes hydroxyle d'alcools n'ayant pas réagi, qui est le produit de la réaction d'estérification d'un alcool à viny!e éther terminal de la formule $R_1CH=CR_2OXOH$ ou d'un mélange d'alcools contenant la proportion d'une mole d'alcool à vinyle éther terminal de la formule susmentionnée et une proportion allant jusqu'à cent moles d'au moins un diol de la formule $Z(OH)_2$, avec une quantité suffisante d'un acide polycarboxylique ayant au moins trois groupes carboxyle, de manière à ce qu'il n'y ait sensiblement aucun groupe hydroxyle d'alcool libre dans l'ester résultant où

   $R_1$ et $R_2$ sont choisis indépendamment parmi l'hydrogène et les groupes alkyle ayant jusqu'à 10 atomes de carbone;

et X et Z sont choisis indépendamment parmi l'alcylène de la formule générale $C_nH_{2n}$, où n est un nombre entier entre 2 et 10;
le poly(éthylèneoxy) ou le poly(propylèneoxy) ayant la formule

$$[-CH_2CH_2O]_m-CH_2CH_2- \quad \text{ou} \quad [-\underset{CH_3}{\underset{|}{CH}}-CH_2O]_m-\underset{CH_3}{\underset{|}{CH}}-CH_2-$$

respectivement, où m est un nombre entre 0 et 49, et un groupe cycloaliphatique de la formule

$[-(CH_2)_n]_2R_3$,

où n est un nombre entier entre 1 et 6 et $R_3$ est un groupe cycloalcylène dont le cycle est un cycle saturé ayant de 5 à 8 carbones.

2. Oligomère selon la revendication 1, caractérisé en ce que X est un alcylène de la formule $-(CH_2)_n-$, où n est un nombre entier de 1 à 4.

3. Oligomère selon la revendication 1, caractérisé en ce que X est un poly(éthylèneoxy) ou un poly-(propylèneoxy) de la formule indiquée dans la revendication 1, où m est de 1 à 5.

4. Oligomère selon la revendication 1, caractérisé en ce que X est un groupe de la formule $-CH_2-R_3-CH_2-$, où $R_3$ est le cyclopentylène, le cyclohexylène, le cycloheptylène ou le cyclooctylène.

5. L'oligomère à vinyle éther terminal de la revendication 1, où l'acide polycarboxylique est un acide tricarboxylique.

6. L'oligomère à vinyle éther terminal de la revendication 1, où l'acide polycarboxylique est un acide tétracarboxylique.

7. Un produit polymérique que l'on peut obtenir par polymérisation de l'oligomère à éther vinylique terminal selon l'une quelconque des revendications 1 à 6.

8. Produit polymérique selon la revendication 7, caractérisé en ce que l'oligomère à éther vinylique terminal contient un divinyle éther.